Europäisches Patentamt

(19) European Patent Office    (11) Publication number:    **0 157 621**
Office européen des brevets                                      **A2**

(12)                              **EUROPEAN PATENT APPLICATION**

(21) Application number: **85302259.8**        (51) Int. Cl.⁴: **A 61 F 2/76**

(22) Date of filing: **01.04.85**

(30) Priority: **04.04.84 GB 8408661**

(43) Date of publication of application: **09.10.85**
Bulletin **85/41**

(84) Designated Contracting States: **AT BE CH DE FR GB IT LI LU NL SE**

(71) Applicant: **UNIVERSITY COLLEGE LONDON, Gower Street, London WC1E 6BT (GB)**

(72) Inventor: **Wilkinson, Derek James, 61 Farleigh Road, New Haw Weybridge Surrey (GB)**
Inventor: **Coombes, Allan Gerald Arthur, 27 Coverts Road, Claygate Esher Surrey (GB)**

(74) Representative: **Boon, Graham Anthony et al, Elkington and Fife High Holborn House 52/54 High Holborn, London, WC1V 6SH (GB)**

(54) **Prosthetic alignment device.**

㉗ There is provided a prosthetic alignment device being composed substantially of plastics material and comprising a first body member (2), a plurality of columns (3) fitted in the first body member, a support member (4) adjustably mounted on each column, a plurality of spacers (5) arranged to fit between the support members and the first body member, and a second body member (6) supported on the support members. The spacers may preferably snap onto the columns.

- 1 -

## PROSTHETIC ALIGNMENT DEVICE

The present invention relates to a prosthetic alignment device,

An alignment device fitted between the socket and the shin-tube of a below-knee prosthesis is discussed below for convenience.

Alignment devices allow the optimum geometrical relationship to be established between the socket and the shin-tube. Adjustment in two perpendicular planes, as well as horizontally, is generally required. Alignment devices may be "built-in" or "temporary".

An example of a built-in device is the "Winnipeg Wedge Disc" system which uses a pair of wedge-shaped discs which are rotatable relative to one another and are fixed between the socket and the shin-tube by a central bolt. Rotation of the discs provides adjustment of the orientation of the shin-tube relative to the socket. This device has the disadvantage that adjustment in one plane affects the adjustment in the other plane and therefore it is often difficult to obtain a specific alignment. Being a built-in device, the disc system does allow adjustment of the prosthesis subsequent to fitting on the patient. However, when the central bolt is released, the discs tend to move from their previous position, thus making controlled adjustment difficult.

An example of a temporary alignment device is the Staros-Gardner which is removed using a duplication jig after alignment and prior to construction of the final prosthesis. The device is made of metal and comprises a pair of plates, one plate being mounted on four screws fitted in the other plate at 90° to one another. Adjustment of two opposite screws provides a change of orientation in one plane, while adjustment of the other two provides a change in the other, perpendicular plane. Since this device is removed after alignment, no subsequent adjustment of the prosthesis is possible.

According to the invention there is provided a prosthetic alignment device being composed substantially of plastics material and comprising a first body member, a plurality of columns fitted in the first body member, a support member adjustably mounted on each column, a plurality of spacers arranged to fit between the support members and the first body member, and a second body member supported on the support members.

The invention has the advantage that, being made of plastics, it is light and corrosion resistant. It can therefore be used as a built-in alignment device, allowing subsequent alignment of the prosthesis.

The spacers provide a calibration of the alignment device as well as forming a load-bearing support between the support members and the first body member. When the device is loosened for re-alignment, the previous alignment is maintained.

Preferably, the spacers can be snapped onto the columns. This means that the device does not have to be dismantled after the alignment has been established.

Advantageously, means to rotate the first and second body members relative to a third member is provided.

Preferably, the first and second body members are plates adapted to be connected to parts of the prosthesis.

Embodiments of the invention are described in detail below, by example only, with reference to the accompanying drawings, wherein:

Fig. 1 is a part-sectional view and part elevation of the prosthetic alignment device;

Fig. 2 is a partly cut-away top plan view of the device; and

Fig. 3 is a side elevation of two of the devices in place in a below-knee prosthesis.

The illustrated device, generally indicated by the reference numeral 1, comprises a first body member 2, several columns 3 fitted in the first member 2, support members 4 adjustably mounted on the columns 3, several spacers 5 fitted between the support members 4 and the first body member 2, and a second body member 6 supported on the support members 4. The parts of the device are composed of a plastics material, preferably a thermoplastics material or a thermoset. The material may be, for example, nylon 6.6.

The first body member comprises a circular plate having a central bore 7. The plate also has four holes 8 arranged around the central bore 7 at 90° spacings. Each hole 8 opens into a slot 9 in the bottom of the plate 2.

A column 3 is press-fitted into each slot 9 and into hole 8. The portion of the column 3 extending above the plate 2 is threaded.

The support members 4 are in the form of cylindrical nuts threaded on the columns 3. Each nut 4 has a domed upper surface 10 and a number of slots 11 around its side.

The spacers 5 are in the form of circular washers which fit around the threaded columns 3. The washers 5 preferably have slots 12 so that they can snap onto the columns 3 without the removal of the nuts 4. A selection of spacers of different thicknesses are provided.

The second body member 6 comprises a circular plate of a similar diameter to plate 2. The plate 6 has four holes 13 through which the columns 3 protrude. The holes 13 have concave seats 14 corresponding to the convex heads of nuts 4; the engagement of the nuts 4 in the seats 14 provides resistance to torque loading on the alignment device. Plate 6 also has a central bore 15 of similar size to the bore 7 of plate 2. Two cylindrical boses 16 are provided on plate 6 to engage in corresponding recesses in, for example, a socket 17. The top of plate 6 is concave to match the convex bottom surface of socket 17.

Beneath the plate 2 is fitted a mounting plate 18 which has a square boss 19 for engagement in a corresponding recess in, for example, a shin-tube 20. The mounting plate 18 has a central bore 21 corresponding to bore 7 of plate 2. The plates 2 and 18 engage each other by means of an annular arrangement of radially extending tongues and grooves, illustrated at 22. The ribs and grooves 22 allow the plate 2 to rotate by a predetermined amount relative to mounting plate 18.

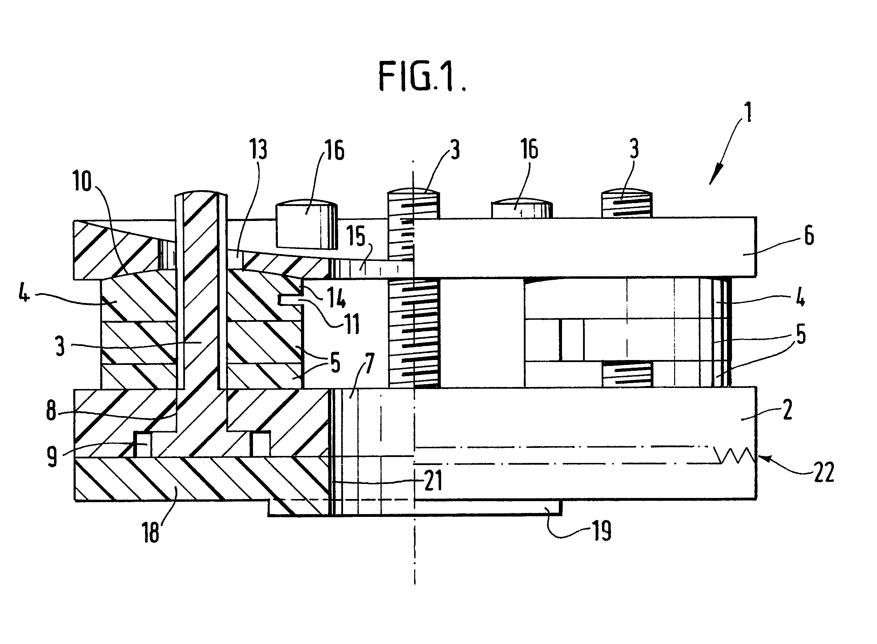

Fig. 1 also shows two of the columns 3 without their nuts and spacers (at the centre of the drawing).

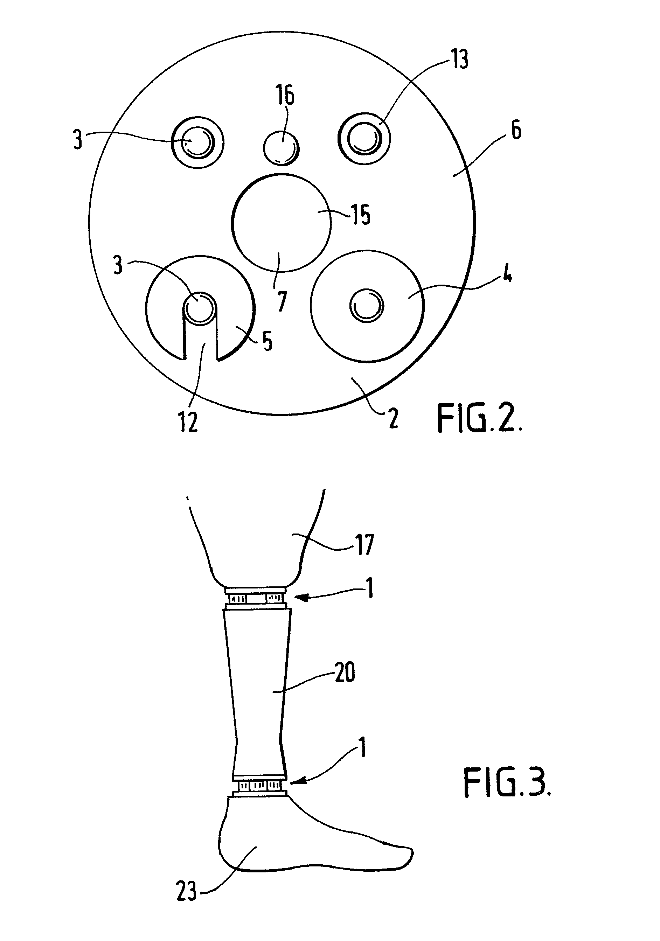

The top part of fig. 2 shows the plate 6 and the bottom half the plate 2. Columns 3 are seen protruding through holes 13 in plate 6. A spacer 5 with its slot 12, nut 4 and central bore 7,15 are also seen in this figure.

The prosthesis of fig. 3 includes socket 17, shin-tube 20 and foot member 23. The shin-tube may particularly be rotationally moulded. An alignment device 1 is connected between the socket 17 and the shin-tube 20 and between the shin-tube 20 and the foot member 23. The connection is made by a bolt 24 (not shown) extending from the socket 17 or foot member 23 into the shin-tube 20 through the central bore 7,15 of the device 1. The alignment device 1 shown in fig. 3 has no mounting plate 18 and therefore the plate 2 is directly attached to the shin-tube 20 or foot member 23, for example via a square boss on its lower surface corresponding to the boss 19 of the mounting plate 18.

To align the prosthesis, the central connecting bolt 24 is loosened. Opposite pairs of nuts 4 are raised or lowered by turning them using an Allen key engaging in the slot 11. This movement tilts the plate 6 and provides adjustment in two perpendicular planes. When the required adjustment has been achieved, the gap between each nut 4 and the plate 2 is filled with one or more spacers 5. Spacers of a suitable thickness are chosen to completely fill the gap. The bolt 24 is then tightened up again.

If the mounting plate 18 is provided, the alignment device and socket can be rotated by a given amount by moving the plate 2 around a given number of the tongues or grooves 22.

The spacers provide a load-bearing support between the nut 4 and the plate 2 so that the column 3 itself does not support the load. Since the device is made of plastics, the threaded engagement (or other means by which the nuts are adjustably supported on the columns) between the nuts 4 and the columns 3 is not itself strong enough to support the weight of the patient.

The spacers provide a clear calibration of the alignment since the prosthetist can quickly see the number and size of spacers on each column. The spacers may each be a given number of millimeters thick. The difference in thickness of spacers on opposite rods can immediately be translated into a given tilt angle in each plane. The entire length of the prosthesis may easily be adjusted by adding a spacer of equal thickness to each column.

A particular advantages of the described alignment device is its low overall height. Its simple design and assembly offers the potential for low cost manufacture by injection moulding or batch production by machining.

- 8 -

It should be noted that the body members of the invention need not be in the form of plates; they may be parts of the prosthesis itself.

CLAIMS

1.      A prosthetic alignment device comprising a first body member (2), a plurality of columns (3) fitted in the first body member, a support member (4) adjustably mounted on each column, and a second body member (6) supported on the support members, characterised in that the device is composed substantially of plastics material and that spacers (5) are provided between the support members and the first body member.

2.      A device according to claim 1, wherein the spacers (5) can be snapped onto the columns (3).

3.      A device according to claim 1 or 2, wherein means to rotate the first and second body members (2,6) relative to a third member (18) is provided.

4.      A device according to claim 3, wherein the third member (18) fits with the first or second body member by means of an annular arrangement of radial interlocking tongues and grooves (22).

5.      A device according to claim 4, wherein the third member is integral with the foot member (23), shank (20) or socket (17) of the prosthesis.

6.      A device according to claim 1 or 2, wherein the first and second body members (2,6) are adapted to be connected to parts of the prosthesis (17,20,23).

# FIG.1.

0157621

FIG.2.

FIG.3.